# EUROPEAN PATENT APPLICATION

(11) **EP 3 991 715 A1**
(43) Date of publication of application: **04.05.2022**
(21) Application number: 21203131.4
(22) Date of filing: 18.10.2021
(51) Int. Cl.: A61K 8/37, A61K 8/31, A61K 8/81, A61Q 5/12, A61Q 5/06

(54) **OIL-BASED COMPOSITION FOR KERATIN FIBERS**

(30) Priority: 30.10.2020 EP 20204935
(71) Applicant: Kao Corporation, Chuo-ku Tokyo 103-8210 (JP)
(72) Inventor: Ebner, Frank, 64297 Darmstadt (DE); Burgdorf, Kristin, 64297 Darmstadt (DE); Aechtner, Anja, 64297 Darmstadt (DE)
(74) Representative: Hoffmann Eitle

(57) **Abstract**

The present invention is directed to a cosmetic composition for keratin fibers comprising: one or more volatile non-silicone lipophilic compound(s) being liquid at 25°C and atmospheric pressure, one or more non-volatile glycerol triester(s) being esterified with one or more linear or branched, saturated or unsaturated C₇ fatty acid(s), one or more non-volatile non-silicone lipophilic compound(s) being liquid at 25°C and atmospheric pressure, and being different from the group above, and one or more thickening agent(s).

## Description

### Field of the invention

The present invention is directed to oil-based compositions for keratin fibers, preferably to conditioning compositions for human hair.

### Background of the invention

Silicone-free hair conditioning products receive more attention from customers, as they are perceived as more sustainable. Silicone-based hair oils usually comprise mixtures of volatile and non-volatile organopolysiloxanes. The volatile components evaporate quickly, whereas the non-volatile counterparts remain on hair and confer a shiny and smooth look. However, the disadvantage of such non-volatile components is that they weight down the hair and leave it without volume.

EP3016630, WO2019/063823 disclose oily compositions to harvest this property for frizz reduction and straightening of hair. Volume reduction appears to be immanent to the effect of the aforementioned prior art. However, the present invention is directed to the opposite effect, namely volume increase.

Hence, there is a need to develop oil-based compositions, which confer excellent conditioning properties to hair while not leaving a weigh-down, flat and volume-less look.

### Summary of the invention

Therefore, the first object of the present inventions is an oil-based cosmetic composition for keratin fibers, preferably for human keratin fibers, more preferably for human hair, comprising:
a) one or more volatile non-silicone lipophilic compound(s) being liquid at 25°C and atmospheric pressure,
b) one or more non-volatile glycerol triester(s) being esterified with one or more linear or branched, saturated or unsaturated C₇ fatty acid(s),
c) one or more non-volatile non-silicone lipophilic compound(s) being liquid at 25°C and atmospheric pressure, and being different from the group according to b),
d) one or more thickening agent(s),
wherein the composition comprises less than 10% by weight of water, calculated to the total weight of the composition.

A second object of the present invention is a method for conditioning of keratin fibers, preferably human keratin fibers, more preferably human hair comprising the steps of:
i) optionally shampooing the keratin fibers,
ii) applying the composition as defined above onto wet or dry keratin fibers, preferably to the lengths of the keratin fibers, and leaving it for a time period in the range of 1 min to 24 h,
iii) optionally washing and shampooing the keratin fibers.

The third object of the present invention is a method for treating split-ends of keratin fibers, preferably human keratin fibers, more preferably human hair, comprising the steps of:
x) applying the composition as defined above onto keratin fibers, preferably onto the dry tips of the keratin fibers,
xi) applying heat to the keratin fibers in a temperature range of 40°C to 230°C, preferably in the range of 60°C to 110°C for a time period in the range of 10 s to 600 s,
xii) optionally washing and shampooing the keratin fibers.

### Detailed description of the invention

Inventors of the present invention have unexpectedly found that the combination of compounds listed in claim 1 provides for an oil-based composition, which has excellent conditioning properties represented by touch, feel, shine and healthy hair appearance. In addition, the composition is lighter leading to a reduced weigh down effect and increased hair volume.

### Composition

The present invention is directed to an oil-based cosmetic composition for keratin fibers, preferably for human keratin fibers, more preferably for human hair, comprising:
a) one or more volatile non-silicone lipophilic compound(s) being liquid at 25°C and atmospheric pressure,
b) one or more non-volatile glycerol triester(s) being esterified with one or more linear or branched, saturated or unsaturated C₇ fatty acid(s),
c) one or more non-volatile non-silicone lipophilic compound(s) being liquid at 25°C and atmospheric pressure, and being different from the group according to b),
d) one or more thickening agent(s),
wherein the composition comprises less than 10% by weight of water, calculated to the total weight of the composition.

The term 'oil-based composition' denotes a composition, which is mainly made of compounds being lipophilic, and, therefore, not water miscible at 25°C and atmospheric pressure without the addition of surfactants other suitable means. Typically, they exhibit an octanol-water coefficient of 0.5 or higher, preferably 1.0 or higher.

It is preferred from the viewpoint of drying speed and composition stability that the composition of the present invention comprises less than 1% by weight of water, calculated to the total weight of the composition, further more preferably it is anhydrous.

It is preferred from the viewpoint of cosmetic applicability that the composition of the present invention is a hair oil.

It is further preferred from the viewpoint of product stability that the composition of the present invention comprises less than 1% by weight of hair dyes and pigments, preferably less than 0.5% by weight of hair dyes and pigments, more preferably less than 0.1% by weight of hair dyes and pigments, calculated to the total weight of the composition, further more preferably it is free of hair dyes and pigments.

The composition of the present invention comprises volatile and non-volatile compounds. The volatility of a compound within the meaning of the present invention is determined as follows:
A drop of the compound is applied to a filter paper and the weight of the immersed filter paper is determined. The filter paper is then stored at 25°C and atmospheric pressure and its weight is re-determined after 24 h.

A compound is non-volatile, if after 24 h 95% by weight or more of the immersed compound is still absorbed by the filter paper.

Hence, volatile compounds evaporate over time and are present at less than 95% by weight after 24 h.

Alternatively, volatile compounds have a nonzero vapor pressure at room temperature and atmospheric pressure, preferably they have a vapor pressure ranging from 0.13 Pa to 40,000 Pa, more preferably they have a vapor pressure ranging from 1.3 Pa to 8000 Pa. Suitable automatic testing can be conducted according to ASTM D5191 ― 01.

### Compound(s) according to a)

The composition of the present invention comprises one or more volatile non-silicone lipophilic compound(s) being liquid at 25°C and atmospheric pressure as compound(s) according to a).

The term 'non-silicone' denotes a volatile oil, which does not contain any silicone atoms.

It is preferred from the viewpoint of volatility that one or more compound(s) according to group a) is a hydrocarbon-based oil having 8 to 16 carbon atoms, preferably it is one or more branched C₁₃ to C₁₅ alkane(s), isododecane, isodecane, and/or isohexadecane, and/or their mixtures, more preferably it is one or more branched C₁₃ to C₁₅ alkane(s).

The most preferred compound(s) according to group a) is available under the INCI name C₁₃ to C₁₅ alkanes.

It is preferred from the viewpoint of hair volume increase that the total concentration of compound(s) according to group a) is 3% by weight or more, more preferably 5% by weight or more, further more preferably 10% by weight or more, still further more preferably 15% by weight or more, still further more preferably 30% by weight or more, calculated to the total weight of the composition.

It is preferred from the viewpoint of hair volume increase that the total concentration of compound(s) according to group a) is 90% by weight or less, more preferably 80% by weight or less, further more preferably 60% by weight or less, still further more preferably 50% by weight or less, calculated to the total weight of the composition.

For attaining the above-mentioned effect, it is preferred that the total concentration of compound(s) according to group a) is in the range of 3% to 90% by weight, preferably in the range of 5% to 80% by weight, further more preferably in the range of 10% to 60% by weight, still further more preferably in the range of 15% to 50% by weight, still further more preferably in the range of 30% to 50% by weight, calculated to the total weight of the composition.

### Compound(s) according to b)

The composition of the present invention comprises one or more non-volatile glycerol triester(s) being esterified with one or more linear or branched, saturated or unsaturated C₇ fatty acid(s) as compound(s) according to group b).

It is preferred from the viewpoint of hair volume increase that one or more compound(s) according to group b) is a glycerol triester with linear or branched, saturated or unsaturated C₇ fatty acid(s), preferably it is triheptanoin.

The term 'glycerol triheptanoate' is synonymous to triheptanoin.

It is preferred from the viewpoint of conditioning effect and volume increase that the total concentration of compound(s) according to b) is 1% by weight or more, more preferably 2% by weight or more, further more preferably 10% by weight or more, still further more preferably 15% by weight or more, calculated to the total weight of the composition.

It is preferred from the viewpoint of conditioning effect and volume increase that the total concentration of compound(s) according to b) is 60% by weight or less, more preferably 55% by weight or less, further more preferably 50% by weight or less, still further more preferably 35% by weight or less, calculated to the total weight of the composition.

For attaining the above-mentioned effects, it is preferred that the total concentration of compound(s) according to group b) is in the range of 1% to 60% by weight, preferably in the range of 2% to 55% by weight, more preferably in the range of 10% to 50% by weight, further more preferably in the range of 15% to 35% by weight, calculated to the total weight of the composition.

It is preferred from the viewpoint of weight-down effect that the weight ratio of compound(s) according to group a) to compound(s) according to group b) is 0.5 or more, more preferably 0.65 or more, further more preferably 1 or more.

It is preferred from the viewpoint of weight-down effect that the weight ratio of compound(s) according to group a) to compound(s) according to group b) is 40 or less, more preferably 25 or less, further more preferably 10 or less.

For attaining the above-mentioned effect, it is preferred that the weight ratio of compound(s) according to group a) to compound(s) according to group b) is in the range of 0.5 to 40, more preferably in the range of 0.65 to 25, further more preferably in the range of 1 to 10.

### Compound(s) according to c)

The composition of the present invention comprises one or more non-volatile non-silicone lipophilic compound(s) being liquid at 25°C and atmospheric pressure, and being different from the group according to b).

It is preferred from the viewpoint of conditioning performance that the compound according to group c) is a natural and/or vegetable oil, petrolatum-based products, C8-C12 fatty alcohols, fatty acids, fatty acid esters comprising C3 to C22 alcohols being esterified with C12 to C22 fatty acids, and polymerization products of butane or isobutene, and/or their mixtures.

Suitable natural and/or vegetable oils are, for example, olive oil, sunflower oil, rapeseed oil, wheatgerm oil, or almond oil.

Suitable fatty acid esters having of linear or branched, saturated or unsaturated fatty acids with C₁₂ to C₂₂ being esterified with linear or branched primary alcohols with C₃ to C₂₂ are isopropyl palmitate, octyl palmitate, isocetyl palmitate, octyl stearate, oleyl oleate, myristyl myristate, isoamyl laurate, and/or their mixtures.

Suitable petrolatum-based products are selected from mineral oil, paraffinum perliquidum, paraffinum subliquidum, and/or paraffinum solidum

Suitably, branched or linear, saturated or unsaturated C₁₂-C₂₂ fatty alcohols are lauryl alcohol, tridecyl alcohol, myristyl alcohol, pentadecyl alcohol, cetyl alcohol, palmitoleyl alcohol, heptadecyl alcohol, stearyl alcohol, oleyl alcohol, nonadecyl alcohol, arachidyl alcohol, behenyl alcohol, and/or their mixtures. Such a mixture is cetearyl alcohol comprising stearyl alcohol and cetyl alcohol.

Suitably, polymerization products of butane or isobutene are homopolymers or copolymers with at least 10 monomers.

It is preferred from the viewpoint of conditioning properties that the molar mass of the polymerization products of butane or isobutene is 750 g/mol or higher, preferably 1,000 g/mol or higher, and 20,000 g/mol or lower.

The most preferred compound according to group c) is hydrogenated polyisobutene, which is partially or fully hydrogenated.

It is preferred from the viewpoint of conditioning performance that the total concentration of compound(s) according to group c) is 10% by weight or more, more preferably 15% by weight or more, further more preferably 25% by weight or more, calculated to the total weight of the composition.

It is preferred from the viewpoint of conditioning performance that the total concentration of compound(s) according to group c) is 90% by weight or less, more preferably 60% by weight or less, further more preferably 50% by weight or less, calculated to the total weight of the composition.

For attaining the above-mentioned effects, it is preferred that the total concentration of compound(s) according to group c) is in the range of 10% to 90% by weight, preferably in the range of 15% to 60% by weight, further more preferably in the range of 25% to 50% by weight, calculated to the total weight of the composition.

It is further preferred from the viewpoint of weight-down effect that the weight ratio of compound(s) according to group a) to compound(s) according to group c) is 0.005 or more, preferably 0.1 or more, further preferably 0.4 or more.

It is further preferred from the viewpoint of weight-down effect that the weight ratio of compound(s) according to group a) to compound(s) according to group c) is 10 or less, preferably 5 or less, further preferably 2 or less.

For attaining the above-mentioned effect, it is preferred that the weight ratio of compound(s) according to group a) to compound(s) according to group c) is in the range of 0.005 to 10, more preferably in the range of 0.1 to 5, further more preferably in the range of 0.4 to 2.

### Compound(s) according to d)

The composition of the present invention comprises one or more thickening agent(s) as compound(s) according to d).

Suitably, the thickening agent(s) according to d) is/are thickening agents being soluble in or mixable with the composition of the present invention.

The term 'soluble' within the meaning of the present invention denotes the that the compounds according to d) may be solid at 25°C and atmospheric pressure, but are able to completely dissolve in the composition of the present invention.

The term 'mixable' denotes that the compounds according to d) may be liquid at 25°C and atmospheric pressure. Hence, the compounds according to d) must be at least partially mixable with the composition of the present invention.

It is preferred from the viewpoint of cosmetic safety that the thickening agent according to d) is an oil-soluble thickening polymer, preferably a non-ionic, anionic, non-ionic, and/or cationic oil-soluble thickening polymer, more preferably a non-ionic oil-soluble polymer.

Hence, it is preferred for the purpose of the present invention that the compound(s) according to d) are able to thicken the composition of the present invention at room temperature of 25°C. Thus, it is preferred from the viewpoint of cosmetic safety of the composition that the viscosity of the composition is 500 mPas or more, more preferably 1,000 mPas or more, further more preferably 2,000 mPas or more, measured by plate-cone viscometry, for example with a Brookfield viscometer with appropriate spindle.

It is preferred from the viewpoint of oil solubility of the thickening polymer that the compound(s) according to d) is a homopolymer or copolymer comprising monomer units of ethylene and/or propylene and/or butylene and/or styrene.

Suitable non-ionic polymers satisfying the description of above are, for example, copolymers of ethylene/butylene with styrene monomers are commercially available under the trade name Kraton polymers, for example Kraton G1701E.

It is preferred from the viewpoint of cosmetic safety that the total concentration of one or more compound(s) according to group d) is 1% by weight or more, preferably 2% by weight or more, further preferably 3% by weight or more, calculated to the total weight of the composition.

It is preferred from the viewpoint of convenience of use that the total concentration of one or more compound(s) according to group d) is 20% by weight or less, preferably 12% by weight or less, further preferably 10% by weight or less, calculated to the total weight of the composition.

For attaining the above-mentioned effect it is preferred that the total concentration one or more compound(s) according to group d) is in the range of 1% to 20% by weight, preferably in the range of 2% to 12% by weight, further more preferably in the range of 3% to 10% by weight, calculated to the total weight of the composition.

### Method of conditioning keratin fibers

The present invention is also directed to a method for conditioning of keratin fibers, preferably human keratin fibers, more preferably human hair comprising the steps of:
i) optionally shampooing the keratin fibers,
ii) applying the composition as defined above onto wet or dry keratin fibers, preferably to the lengths of the keratin fibers, and leaving it for a time period in the range of 1 min to 24 h,
iii) optionally washing and shampooing the keratin fibers.

Step i) is optional and based on the taste of the consumer.

In one aspect of the present invention, the composition is left on keratin fibers for a time period of 1 min to 60 min in step ii). This time period is preferred if the composition is applied as hair mask, and then preferably be rinsed-off and shampooed in step iii).

In another aspect of the present invention, the composition is left on keratin fibers for a longer period, for example overnight. In such cases the time period of step ii) is in the range of 1 h to 24 h. Then the washing and shampooing of step iii) remains optional according to the taste of the consumer.

### Method of treating split ends

The present invention is also directed to a method for treating split-ends of keratin fibers, preferably human keratin fibers, more preferably human hair, comprising the steps of:
x) applying the composition as defined above onto keratin fibers, preferably onto the dry tips of the keratin fibers,
xi) applying heat to the keratin fibers in a temperature range of 40°C to 230°C, preferably in the range of 60°C to 110°C for a time period in the range of 10 s to 600 s,
xii) optionally washing and shampooing the keratin fibers.

In one aspect of the present invention, the keratin fibers are heated in the temperature range from 100°C to 230°C as prescribed in step xi). Suitably heating means are, for example, flatirons and round irons. Suitably time periods for heating are in the range of 10 s to 120 s per streak, as prescribed in step xi).

In another aspect of the present invention, keratin fibers are treated with lower heat in the range of 40°C to 110°C, preferably 60°C to 110°C, as prescribed in step xi). Suitable heating means is a blow dryer. Heat exposure may be extended for a time period in the range of 10 s to 600 s, as prescribed in step xii).

### EXAMPLES

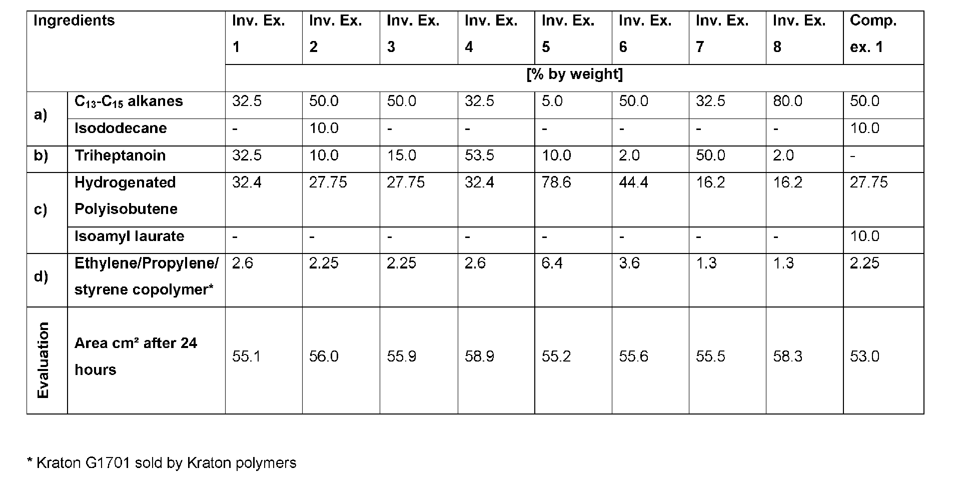

### Methods

Caucasian hair streaks (2 g hair per streak) were shampooed with a commercially available shampoo under the brand name Goldwell Dualsenses Scalp Specialist Deep Cleansing Shampoo, dried and applied 0.3 g of the compositions from above. The streaks were then placed in a closed chamber with controlled atmosphere for 24 h (50% relative humidity, 25°C).

Each hair streak was placed in a freely-hanging fashion and combed through for three times with a conventional hair dresser comb. Photos for each hair streak were taken from a specified distance, which were then analyzed by a proprietary image analysis software by calculating the occupied area by the hair streak in the image based on the grey scale contrast differences between the hair streaks and the background. The area was recorded as cm².

Lower area values correspond to lower hair volumes.

## Claims

1. An oil-based cosmetic composition for keratin fibers, preferably for human keratin fibers, more preferably for human hair, comprising:
a) one or more volatile non-silicone lipophilic compound(s) being liquid at 25°C and atmospheric pressure,
b) one or more non-volatile glycerol triester(s) being esterified with one or more linear or branched, saturated or unsaturated C₇ fatty acid(s),
c) one or more non-volatile non-silicone lipophilic compound(s) being liquid at 25°C and atmospheric pressure, and being different from the group according to b),
d) one or more thickening agent(s),
wherein the composition comprises less than 10% by weight of water, calculated to the total weight of the composition.

2. The composition according to claim 1 **characterized in that** it comprises less than 1% by weight of water, calculated to the total weight of the composition, further more preferably it is anhydrous.

3. The composition according to any of the claims 1 and/or 2 **characterized in that** one or more compound(s) according to group a) is a hydrocarbon-based oil having 8 to 16 carbon atoms, preferably it is one or more branched C₁₃ to C₁₅ alkane(s), isododecane, isodecane, and/or isohexadecane, and/or their mixtures, more preferably it is one or more branched C₁₃ to C₁₅ alkane(s).

4. The composition according to any of the preceding claims **characterized in that** the total concentration of compound(s) according to group a) is in the range of 3% to 90% by weight, preferably in the range of 5% to 80% by weight, more preferably in the range of 10% to 60% by weight, further more preferably in the range of 15% to 50% by weight, still further more preferably in the range of 30% to 50% by weight, calculated to the total weight of the composition.

5. The composition according to any of the preceding claims **characterized in that** one or more compound(s) according to group b) is a glycerol triester with linear or branched, saturated or unsaturated C₇ fatty acid(s), preferably it is triheptanoin.

6. The composition according to any of the preceding claims **characterized in that** the total concentration of compound(s) according to group b) is in the range of 1% to 60% by weight, preferably in the range of 2% to 55% by weight, more preferably in the range of 10% to 50% by weight, further more preferably in the range of 15% to 35% by weight, calculated to the total weight of the composition.

7. The composition according to any of the preceding claims **characterized in that** one or more compound according to group c) is a natural and/or vegetable oil, petrolatum-based products, C8-C12 fatty alcohols, fatty acids, fatty acid esters comprising C3 to C22 alcohols being esterified with C12 to C22 fatty acids, and polymerization products of isobutene, and/or their mixtures.

8. The composition according to any of the preceding claims **characterized in that** one or more compound(s) according to group c) is selected from hydrogenated polyisobutene.

9. The composition according to any of the preceding claims **characterized in that** the total concentration of compound(s) according to group c) is in the range of 10% to 90% by weight, preferably in the range of 15% to 60% by weight, further more preferably in the range of 25% to 50% by weight, calculated to the total weight of the composition.

10. The composition according to any of the preceding claims **characterized in that** one or more compound(s) according to group d) is an oil-soluble thickening polymer, preferably it is a homopolymer or copolymer comprising monomer units of ethylene and/or propylene and/or butylene and/or styrene.

11. The composition according to any of the preceding claims **characterized in that** the total concentration one or more compound(s) according to group d) is in the range of 1% to 20% by weight, preferably in the range of 2% to 12% by weight, further more preferably in the range of 3% to 10% by weight, calculated to the total weight of the composition.

12. The composition according to any of the preceding claims **characterized in that** it is a hair oil.

13. The composition according to any of the preceding claims **characterized in that** it comprises less than 1% by weight of hair dyes and pigments, preferably less than 0.5% by weight of hair dyes and pigments, more preferably less than 0.1% by weight of hair dyes and pigments, calculated to the total weight of the composition, further more preferably it is free of hair dyes and pigments.

14. A method for conditioning of keratin fibers, preferably human keratin fibers, more preferably human hair comprising the steps of:
i) optionally shampooing the keratin fibers,
ii) applying the composition as defined in the claims 1 to 13 onto wet or dry keratin fibers, preferably to the lengths of the keratin fibers, and leaving it for a time period in the range of 1 min to 24 h,
iii) optionally washing and shampooing the keratin fibers.

15. A method for treating split-ends of keratin fibers, preferably human keratin fibers, more preferably human hair, comprising the steps of:
x) applying the composition as defined in the claims 1 to 13 onto keratin fibers, preferably onto the dry tips of the keratin fibers,
xi) applying heat to the keratin fibers in a temperature range of 40°C to 230°C, preferably in the range of 60°C to 110°C for a time period in the range of 10 s to 600 s,
xii) optionally washing and shampooing the keratin fibers.
